# EUROPEAN PATENT APPLICATION

(11) **EP 1 138 279 A2**
(43) Date of publication of application: **04.10.2001**
(21) Application number: 01106798.0
(22) Date of filing: 19.03.2001
(51) Int. Cl.: A61F 2/06

(54) **Improved stent for dilatation of blood vessels**

(30) Priority: 23.03.2000 IT MI000611
(71) Applicant: Cioffi, Antonio Mirko, 20016 Pero (MI) (IT)
(72) Inventor: Cioffi, Antonio Mirko, 20016 Pero (MI) (IT)
(74) Representative: Petruzziello, Aldo

(57) **Abstract**

A stent (1) for dilation of blood vessels, such as arteries (10) and the like, comprises a tubular net (2) at the ends of which are two annular elements (5, 6), said stent being suitable to pass from a collapsed configuration in which it assumes a minimum cross sectional diameter in order to be able to be conveyed inside the artery (10) to an expanded configuration in which it assumes a maximum cross-sectional diameter in order to dilate the walls of the part of artery in which it is inserted and keep them in this dilated position; in the expanded configuration the annular elements (5,6) compressing the inner walls (11) of the artery (10) so as not to create any breaks or interruptions in the flow of blood inside the artery (10) and through the stent.

## Description

The present invention refers to an improved stent for dilation of blood vessels, in particular arteries.

In the coronary arteries which carry the blood directly to the heart, it can happen that deposits of fat or cholesterol gradually form, due to factors such as genetic predisposition, diabetes, smoking, high-fat diet, and high blood pressure (hypertension). These fatty deposits in the arteries form occlusions or strictures called stenoses which are capable of limiting blood flow.

If the necessary amount of blood does not reach the heart, serious problems can occur such as cardiac ischaemia, that is lack of oxygen in the cardiac muscle, and so called angina pectoris, perceived by the patient as chest pain. If the fatty deposits form an actual plug in the artery, blood can no longer reach the heart and myocardial infarction occurs.

When a patient has an abnormal electrocardiogram in an exercise tolerance test, a specific examination called a coronary angiography must be performed to see if the abnormal electrocardiogram is due to occlusion of an artery that carries blood to the heart. The coronary angiography consists in injecting a radio-opaque, iodine-based contrast agent into the arteries through a catheter. When X-rays strike the contrast agent an image is formed on a cine film. The physician can thus see a film that shows the flow of blood through the arteries and the presence of any of the narrowings or strictures of blood flow that indicate stenosis.

Various methods of solving the problem of stenoses are known at present.

One method is the by-pass. This is a surgical intervention by means of which an alternative route is created for the blood flow in the point of the artery in which the occlusion has occurred. This means using a leg vein and a thoracic artery from the patient to fashion an alternative artificial duct for the blood in the point of the artery in which the narrowing or occlusion has occurred. It is evident that a by-pass is a somewhat complicated surgical procedure. It involves opening the patient's chest, therefore it is done under general anaesthesia and the patient is subjected to be bedfast for long periods.

An alternative to a by-pass is an angioplasty, which allows the narrowings or stenoses present in the coronary arteries to be eliminated or widened without resorting to surgical opening of the patient's chest and general anaesthesia. To eliminate these narrowings, use is made of devices such as a micro drill or laser; to widen the strictures a balloon system is used.

In angioplasty procedures a catheter is used, that is to say a small, hollow tube that is generally introduced into the patient's femoral artery or aorta. A small metal guide wire is inserted through this catheter and manoeuvred in the desired direction so that its tip reaches the artery affected by the stenosis which requires attention. According to requirements, a micro drill, a laser device or an inflatable balloon can be installed on the guide wire.

If the narrowing of the arteries is due to deposits of calcified and therefore extremely hard fat, the micro drill, which consists of an oval body on the surface of which diamond granules are provided to make it abrasive, is used. The micro drill is brought close to the parts of occluded artery and made to rotate at a speed of about 180-200 thousand revolutions per minute, so as to eliminate the calcified fat that causes the narrowing of the arteries. The micro drill produces extremely small fragments, smaller in size than red blood cells, so these fragments can be eliminated in the normal blood circulation.

As an alternative to the micro drill a device that generates a laser beam can be used. This laser beam has an area of action of about a few tenths of a millimetre and by operation of the guide wire the laser beam gradually opens the way through the occluded artery, destroying the deposits of fat that have produced the narrowing.

If the fatty deposits in the arteries are not yet calcified, instead of using such complex devices as the micro drill and the laser, a balloon system is used that has a deflated balloon mounted on the tip of the guide wire. Once the balloon has reached the point of the artery where there is a narrowing, it is inflated and kept expanded for a few seconds. This manoeuvre is repeated two or three times. The object is to compress the deposits of fat which have not yet calcified and are therefore still soft, against the walls of the artery, squeezing them to reduce the space they occupy. In this manner the blood is allowed to flow better in the arteries without encountering obstacles.

In many cases it has happened that the widening of the artery produced by the balloon has not lasted over time. After three or four days or after one or two months the artery closed again like before, as if it were caving in on itself.

To overcome this drawback, according to the prior art use has been made of a stent. The stent is a metal reinforcement in the form of a tubular net. After the artery has been widened with the balloon method, the stent is inserted over the balloon. The balloon is then inflated to expand the stent which abuts against the inside walls of the artery. At this point the balloon is deflated so that it can be removed from the artery together with the guide wire and consequently the stent remains expanded against the artery walls, avoiding any caving in of this area of the artery.

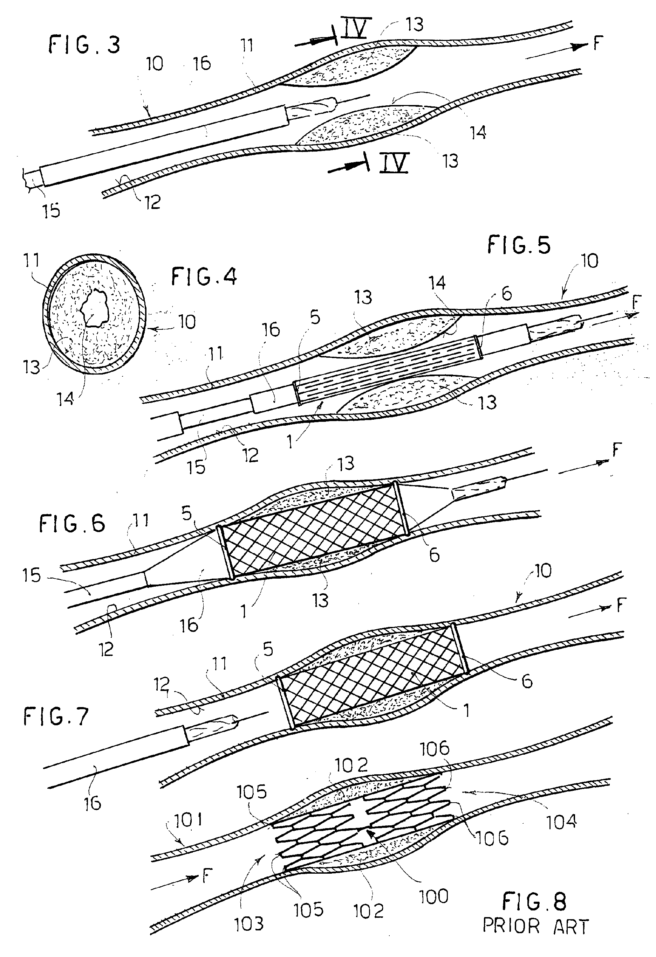

Figure 8 shows diagrammatically a stent 100 according to the prior art, applied to an artery 101 in which fatty plaque 102 which is kept apart by the stent 100 is visible. The stent 100 must be radially expandable in order to be able to pass from an unexpanded configuration in which it occupies a minimum space to an expanded configuration in which it is radially expanded. In fact the stent is inserted in the artery in its unexpanded configuration and, having reached the point in which the stenosis is situated, is expanded radially by means of a balloon to its expanded configuration. Although it has a very limited length, the stent 100 must be substantially flexible so that it can adapt to the shape of the artery. Finally the stent 100 must be substantially rigid in order to be able to maintain its expanded configuration and thus avoid caving in of the parts of artery it supports.

In compliance with these requirements the stent 100 takes the form of a net with a diamond-shaped mesh. At the ends 103 and 104 of the stent 100, between the diamond-shaped meshes and the inner wall of the artery 101, respective steps 105 and 106 are formed.

Considering that the blood flow in the artery 101 proceeds in the direction of the arrow F, said flow encounters a barrier in the steps 105 formed at the end 103 of the stent 100. Consequently the blood platelets break against said steps 105 and accumulate in the entry end of the stent. This favours the formation of a new stenosis which affects the artery 101 near the end 103 of the stent 100.

The object of the present invention is to eliminate said drawback, providing an improved stent that is practical, economical and simple to make.

This object is achieved, according to the invention, with the characteristics listed in appended independent claim 1.

Advantageous embodiments of the invention are apparent from the dependent claims.

The stent according to the invention provides an annular element positioned in at least one of the two ends of the stent. This annular element further widens the artery, compressing the inside walls of the artery with which it is in contact. In this manner no step is formed between it and the inner surface of the artery. With the stent according to the invention blood can flow freely in the artery without encountering any obstacle that causes an interruption in the flow of the platelets and thus favours the formation of accumulations of fat and consequent stenoses.

Further characteristics of the invention will be made clearer by the detailed description that follows, referring to a purely exemplary and therefore non-limiting embodiment thereof illustrated in the appended drawings, in which:
Figure 1 is an axonometric view of a stent according to the invention, shown in an expanded configuration;
Figure 1a is a cross section of an end of the stent according to the invention;
Figure 2 is an axonometric view, showing the stent according to the invention applied to an artery, partially cut away;
Figure 3 is a diagramatic view in longitudinal section showing an artery in which a stenosis has formed, during an angioplasty procedure and before application of a stent;
Figure 4 is a cross section taken along section line of IV-IV of Figure 3;
Figures 5, 6 and 7 are diagramatic views in longitudinal section, showing the procedure for inserting of the stent according to the invention inside the artery of Figures 3 and 4;
Figure 8 is a longitudinal section showing a stent according to the prior art applied to an artery.

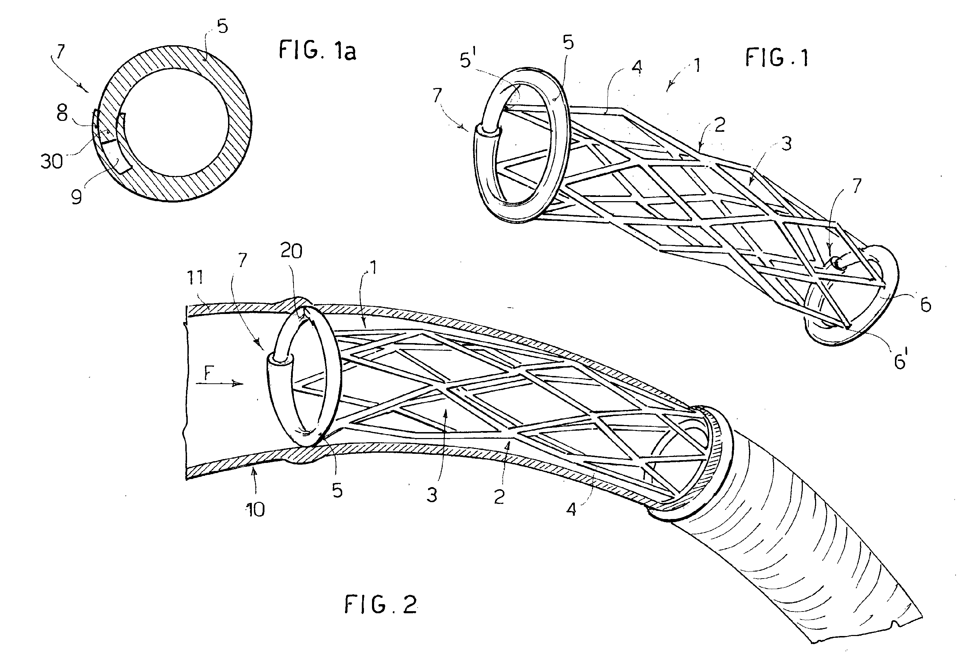

The stent according to the invention, which is designated as a whole with reference numeral 1, is described with the aid of Figures 1 and 1a.

The stent 1, shown in its expanded position, comprises a net 2 with a substantially tubular shape, formed by thin wires 4 which are woven together so as to form a plurality of diamond-shaped meshes 3. The wires 4 are generally made of steel or another precious metal, but they can also be of synthetic material or in any case of a rigid material such as not to cause rejection by the arterial tissue. Thanks to its shape and the choice of the material, the tubular net 2 can pass from a collapsed configuration with a minimal cross-sectional diameter to an expanded configuration with a maximum diameter in cross section. Once it has assumed the expanded configuration, as shown in Figure 1, said configuration is maintained thanks to the stiffness of the wires 4.

At the two ends of the tubular net 2 two annular elements 5 and 6, respectively, are provided. Each annular element 5 and 6 connects the respective end tips 5' and 6' of the diamond-shaped meshes 3. The annular elements 5 and 6 can be made from the same material used for the wires 4 or from a different, substantially rigid material compatible with the use for which the stent is intended. Like the tubular net 2, the annular elements 5 and 6 must be able to pass from a collapsed configuration with a minimum diameter to an expanded configuration with a maximum diameter. For this purpose each annular element 5, 6 has a telescopic closing system, 7.

Figure la shows a cross section of the annular element 5. An end 8 of the annular element 5 has a circular hole 9 having an inside diameter slightly larger than the outside diameter of the other end 30 of the annular element 5. The end 30 can thus engage by sliding telescopically inside the hole 9, so as to close the ring. Stops positioned inside the hole 9 and at the end 30 of the ring element 5 can obviously be provided in the telescopic closing system.

Said stops block the stroke of the end 30 inside the hole 9 in a collapsed position in which the annular element 5 assumes a minimal diameter and in an expanded position in which the annular element 5 assumes a maximum diameter thus avoiding the possibility of opening of the annular element 5.

The method for introducing a stent 1 according to the invention into an artery is described hereunder with reference to Figures 3-7.

Figures 3 and 4 show an artery 10 having a substantially cylindrical shape, generated by an arterial tissue 11 which defines a duct 12 through which the blood flows toward the heart, following the direction of the arrow F.

In an area of said artery there is a stenosis, that is to say a build up of fat 13 which causes a narrowing 14 of the duct 12 through which the blood flows.

In order to widen said narrowing 14, an angioplasty procedure can be carried out with an inflatable balloon. A catheter, formed by a small, hollow tube, is inserted in an artery, for example the femoral artery, of the patient. A guide wire 15 on the tip of which a deflated balloon is installed, is inserted through the catheter. The guide wire 15 is advanced by the surgeon until its tip reaches the narrowing 14 of the artery 10. At this point the balloon 16 is inflated so that it compresses the accumulations of fat 13, widening the stricture 14. The ballon is maintained inflated for few seconds and then it is deflated. This operation of inflating and deflating the balloon is repeated two or three times.

The guide wire 15 is then extracted from the catheter and the stent 1 according to the invention is positioned over the deflated balloon 16, in a collapsed configuration with a minimal diameter that is just sufficient for its insertion over the deflated balloon 16. Then, as shown in Figure 5, the guide wire is brought back inside the artery 10, with its tip at the narrowing 14 which has previously been widened by inflation of the balloon 16.

At this point, as shown in figure 6, the balloon 16 is inflated. Consequently, through the pressure of the balloon 16, the stent 1 passes from its collapsed position to the expanded position and compresses the accumulations of fat 13 in the artery 10, widening the narrowing 14 to a size substantially similar to that of the duct 12 of the artery 10. In particular the ends 30 and 8 of each annular element 5 and 6 slide telescopically inside each other allowing widening of the respective annular elements 5 and 6, so that the annular elements 5 and 6 compress the inside wall 11 of the artery 10.

Afterwards, the balloon 16 is deflated and the stent 1, thanks to its rigid structure, maintains the expanded configuration, thus preventing the accumulations of fat 13 compressed thereby from forming a narrowing in the duct 12 of the artery 10 again. Thus, as shown in Figure 7, the guide wire 15 with the deflated balloon 16 is removed from the artery 10, leaving the stent 1 in its expanded position, preventing caving in of the portion of the artery 10 with the accumulations of fat 13.

As shown better in Figure 2, when the stent 1 is in its expanded position inside the artery 10, the annular elements 5 and 6 positioned at the ends of the tubular net 2 of the stent 1 compress the inner walls 11 of the artery 10, creating an annular seat 20 in which they are housed. In this manner it is possible to minimize the discontinuity between the inner wall of the artery 10 and the annular elements 5 and 6 of the stent 1. Thus the flow of blood in the direction of the arrow F does not encounter any obstacle due to the presence of the annular elements 5 and 6; consequently the blood can flow in the artery 10 through the stent 1 without encountering points of discontinuity in which stagnation of platelets, and thus the formation of a new stenosis, can occur.

Clearly, instead of the two annular elements 5 and 6 at the two ends of the tubular net 2 of the stent, a single annular element 5 can be provided, positioned in the end of the stent, destined to be the end through which the blood flow enters.

Instead of the telescopic closing system of the annular elements 5 and 6, another closing system can be used, such as system of guides, an elastic system or a snap system, able to allow expansion of the annular element 5, 6 starting from a collapsed position.

Various modifications of detail within the reach of a person skilled in the art can be made to the present embodiment of the invention, without thereby departing from the scope of the invention expressed by the appended claims.

## Claims

1. A stent (1) for dilating blood vessels, such as arteries (10) and the like, comprising a tubular net (2) able to pass from a collapsed configuration in which it assumes a minimum cross-sectional diameter in order to be able to be conveyed inside the artery (10) and an expanded configuration in which it assumes a maximum cross-sectional diameter so as to radially dilate the walls of a portion of said artery (10) in which it is inserted and hold them in said dilated position, **characterized in that** at least in one of the ends of said tubular net (2) an annular element (5, 6) is provided with a variable diameter able to follow the tubular net (2) from said collapsed configuration to said expanded configuration, said at least one annular element (5, 6) in said expanded position compressing the inner walls (11) of said artery (10) so as not to create discontinuities or interruptions in the blood flow that flows inside the artery (10) and through the stent (1).

2. A stent according to claim 1, **characterized in that** said at least one annular element (5) with a variable diameter is provided at the end of the stent for entry of the blood flow that flows in the artery (10).

3. A stent according to claim 1, **characterized in that** at each end of said tubular net (2) a respective annular element (5, 6) with a variable diameter is provided.

4. A stent according to claim 1, 2 or 3 **characterized in that** said annular element (5) has closing means (7) able to allow widening of said annular element (5, 6) from said collapsed configuration to said expanded configuration.

5. A stent according to claim 4, **characterized in that** said closing means (7) have a telescopic coupling element between the ends (8, 30) of said annular element (5, 6).

6. A stent according to claim 4 or 5, **characterized in that** said closing means (7) have stop means able to prevent opening of said annular element (5, 6).

7. A stent according to any one of the preceding claims, **characterized in that** said tubular net (1) is formed by wires (4) woven together so as to form substantially diamond-shaped meshes (3), at least some of the tips of said meshes at the ends of the tubular net (2) being connected to said at least one annular element (5, 6).

8. A stent according to claim 7, **characterized in that** said wires (4) of the tubular net (2) and said annular elements (5, 6) are made of steel.

9. A stent (1) according to any one of the preceding claims for use in arteries subject to stenosis.
